# EUROPEAN PATENT APPLICATION

(11) **EP 1 767 210 A1**
(43) Date of publication of application: **28.03.2007**
(21) Application number: 05757963.3
(22) Date of filing: 27.06.2005
(51) Int. Cl.: A61K 31/7048, A61P 25/00, A61P 25/16

(54) **THE MEDICAL USE OF PAEONIFLORIN**

(30) Priority: 02.07.2004 CN 200410025717
(71) Applicant: Shanghai Institute of Materia Medica, Chinese Academy of Sciences, Pudong New District Shanghai 201203 (CN)
(72) Inventor: ZHU, Xingzu, Pudong New District, Shanghai 201203 (CN); LIU, Dazhi, Pudong New District, Shanghai 201203 (CN); YE, Yang, Pudong New District, Shanghai 201203 (CN); LIU, Huaqing, Pudong New District, Shanghai 201203 (CN)
(74) Representative: Grünecker, Kinkeldey, Stockmair & Schwanhäusser Anwaltssozietät
(86) International application number: PCT/CN2005/000929
(87) International publication number: WO 2006/002589

(57) **Abstract**

The present invention relates to the medical use of paeoniflorin with the following structure 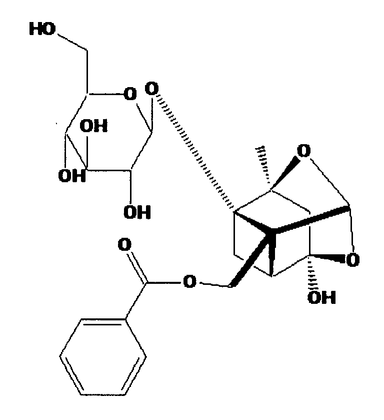 in treating and preventing nervous system diseases, such as apoplexy and Parkinson's disease, etc.

## Description

### Technical Field

The present invention relates to the medical use of paeoniflorin, a selective agonist of non-purine adenosine receptor-1 (A₁R), particularly to the use of the compound treating and preventing nervous system diseases, such as apoplexia and Parkinson's disease, by selectively activating A₁R. And this compound eliminates the serious cardiovascular side effects of the traditional purine A₁R agonists.

### Background Art

Paeoniflorin is a natural active compound derived from the root of Ranunculaceae plants, such as white peony, red peony and peony. Its contents in the source plants are high. For example, the white peony from Cao county, Shan Dong province of China contains up to 1.95% of the active component; what is more, the contents of paeoniflorin in the root of peony reaches up to 7 %. Further, its source plants are of wide geographical distribution and easy to be grown artificially, so that they are suitable to be planted industrially in a large scale. Some pharmacodynamical studies show that this compound has various effects in analgesia, sedation, spasmolysis, anti-inflammation, antiulcer, expanding coronary artery, anti-acute myocardial ischemia, and inhibiting agglomeration of platelet etc., and has a large safe dose range.

It is found in many studies that, on one hand, A₁R receptor agonists can activate the pre-synaptic A₁R receptor to inhibit the release of excitatory amino acids, such as glutamic acid etc., and relieve the excitotoxicity of cells and overloading of Ca²⁺; on the other hand, they can act on the post-synaptic A₁R receptor to increase the conductance of K channels and inhibit the excitation of neuron by inhibiting the depolarization of cellular membrane. Thereby, they have effective protective effect on nerves (Heron A, Lekieffre D, Le Peillet E, Lasbennes F, Seylaz J, Plotkine M, Boulu RG, Effects of an A1 adenosine receptor agonist on the neurochemical, behavioral and histological consequences of ischemia. Brain Res. 1994; 641 (2): 217-24., Goldberg MP, Monyer H, Weiss JH, Choi DW., Adenosine reduces cortical neuronal injury induced by oxygen or glucose deprivation in vitro. Neurosci Lett. 1988; 9(3): 323-7., Mori M, Nishizaki T, Okada Y, Protective effect of adenosine on the anoxic damage of hippocampal slice. Neuroscience. 1992; 46(2): 301-7., Von Lubitz DK, Beenhakker M, Lin RC, Carter MF, Paul IA, Bischofberger N, Jacobson KA., Reduction of postischemic brain damage and memory deficits following treatment with the selective adenosine A1 receptor agonist. Eur J Pharmacol. 1996; 302(1-3): 43-8.). However, due to the serious cardiovascular side effects, the A₁R receptor agonists are greatly limited in the clinical application (Collis MG, Hourani SM., Adenosine receptor subtypes. Trends Pharmacol Sci. 1993; 14(10): 360-6. Review., Daval JL, Nehlig A, Nicolas F., Physiological and pharmacological properties of adenosine: therapeutic implications. Life Sci. 1991; 49(20): 1435-53. Review.). In recent years, great efforts have been made in the international medical field with a large amount of human and material resources to find some selective A₁R receptor agonists which have reliable protective effects on the nerves and little cardiovascular side effects.

Through our study based on the high throughput screening platform of ligand-receptor competitive binding assay, we find for the first time that the paeoniflorin selectively and competitively binds to A₁R. The paeoniflorin has a different chemical structure from traditional A₁R receptor agonists, and is a new non-purine A₁R receptor selective agonist. Through some classical experimental animal models, it is proved that the paeoniflorin has the effects of treating and preventing nervous system diseases, such as apoplexy and Parkinson's disease etc. Further studies demonstrate that it has low cardiovascular side effects. So the paeoniflorin is much valuable to be used clinically.

Nervous system diseases, such as apoplexy and Parkinson's disease etc., are among the greatest threats against the health of people, and the morbidity and disabling rate of nervous system diseases in aged people remain at high level. For example, apoplexy has an incidence rate of about 0.35 % in the people of age between 45-89, and in the apoplectic patients, 20% of them have a survival time less than 1 month; more than 30% of the apoplectic patients having a survival time over 6 months can not take care of themselves. Thus, the patients, their families and the society all bear the heavy burden of mentality and economy.

The present invention provides a novel drug for treating and preventing nervous system disease, such as apoplexy and Parkinson's disease etc., which has great social and economic benefit.

### Disclosure of Invention

The invention discloses that the paeoniflorin is a non-purine A, receptor selective agonist.

And the object of the invention is to provide the use of paeoniflorin in treating and preventing nervous system diseases, such as apoplexy and Parkinson's disease etc.

The present invention extracts and isolates a natural active compound paeoniflorin from roots of Ranunculaceae plants white peony, red peony and peony by some chemical processes. And through some pharmocological assays, it is found that paeoniflorin, as a selective agonist of non-purine type adenosisne receptor-1, may be used in preparation of drugs for treating and preventing nervous system disease, such as apoplexy and Parkinson's disease etc.

The roots of white peony, red peony and peony are used as raw materials in the present invention, and the preparing process is as follows:

The roots of white peony, red peony or peony are extracted by refluxing with ethanol to obtain dry extractum; the extractum is solved completely with water and passed through a macroporous resin; the macroporous resin is eluted with water until the saccharide reaction become negative, and eluted with ethanol of twice column volumes to obtain extractum; the pure compound paeoniflorin is separated by silica gel column chromatography.

The chemical structure of paeoniflorin is as follows: 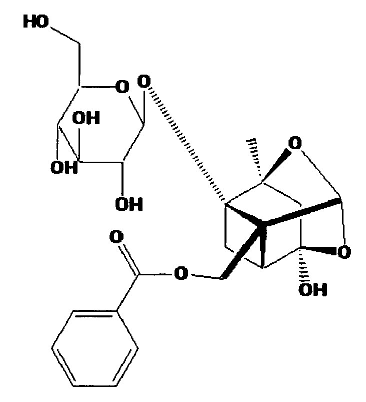 Its molecular formula is: C₂₃H₂₈O₁₁, its molecular weight is 480.48, and its chemical name is:
β-D-Glucopyranoside,56-[(benzoyloxy)methyl]tetrahydro-5-hydroxy-2-methyl-2,5-methano-1H-3,4-dioxacyclobuta [cd] Pentalen-la (2H) -yl, [lar-claα, 2β, 3aα, 5aα, 5bα]].

The extraction and purification method of paeoniflorin can be found in the reference document (JIN Jishu, DU Shuhu, CHONG Mingcai. Separation of total paeoniflorin with macroporous adsorptive resin, Chinese Journal of Chinese Traditional Medicine, 1994. VOL. 19 NO. 1:31).

The present invention obtained the paeoniflorin with the above process and carried out the following pharmacological and pharmacodynamic tests.

### 1. Ligand-receptor competitive binding assay of paeoniflorin:

Synaptic membranes of rat cerebral cortex are employed in the assay. After decapitation of the rat, cerebral cortex is separated and homogenized sufficiently in 1: 15 (W/V) sucrose solution, after centrifuged, the supernatant is taken and centrifuged again. After discarding the supernatant, the sediment is resuspended in 1: 30 (W/V) Tris-HCl buffer (50 mM, PH 7.4), homogenized, washed and then centrifuged, after repeating the wash process 3 times, the sediment is resuspended in 1: 5 (W/V) Tris-HCl buffer (50 mM, PH 7.4) again and subpakaged, stored at -80 °C. The protein concentration is measured by Bradford method (Bradford MM. A rapid and sensitive method for the quantitation of microgram quantities of proteins utilizing the principle of protein-dye binding. Anal Biochem. 1976; 72: 248-254.). All the above procedures are operated at 4°C .

The above model is used to evaluate the competitive binding of paeoniflorin with the A₁R and A₂ₐR receptor mixed agonist [³H]-ethyl amide adenosine ([3H]-NECA) and the A₁R receptor selective agonist [3H]-CCPA.

The experiment proves that the paeoniflorin can establish a competitive binding with [3H]-CCPA, and its competitive binding with the [3H]-NECA shows a typical two-site competition property (EC50₁=13.9nM, EC50₂=5.00µM). The result indicates that the paeoniflorin binds to A₁R and A_{2A}R receptors simultaneously with a higher selectivity (EC50₂/ EC50₁=360), that is, it selectively binds to A₁R at low concentration and binds to A_{2A}R at high concentration.

### 2. Pharmacodynamic assay of paeoniflorin for preventing and treating apoplexy:

The transient and permanent focal cerebral ischemia models are obtained by the method of reversible middle artery occlusion in rat (Belayev L, Alonso OF, Busto R, Zhao W, Ginsberg MD. Middle cerebral artery occlusion in the rat by intraluminal suture. Neurological and pathological evaluation of an improved model. Stroke, 1996; 27: 1616-22., Takano K, Tatlisumak T, Bergmann AG, Gibson DG 3rd, Fisher M. Reproducibility and reliability of middle cerebral artery occlusion using a silicone-coated suture (Koizumi) in rats. J Neurol Sci. 1997; 153: 8-11.) to evaluate the effect of paeoniflorin on the neurological defect physical sign and the cerebral injured volume in cerebral ischemia rat respectively. The rat is anesthetized by intraperitoneal injection of chloralosane, the blood pressure is monitored by a right femoral arterial cannula, and the rectal temperature remains at 36.5-37.5 °C. The cervical part is cut to dissociate the external carotid artery, branch arteries are severed by electrocoagulation, and the distal end is ligated, a monofilament nylon thread of No. 4-0 with the drumstick-like head is inserted from external carotid artery into the anterior cerebral artery via the internal carotid artery to block the initial section of the middle cerebral artery and also block the anterior cerebral artery, the posterior cerebral artery and the branch arteries of the internal carotid artery. For the transient MCAO model group, occlusion thread is drawn out after 1.5 hours and perfusion is performed for 22.5 hours, wherein physiological saline and various doses of paeoniflorin are injected subcutaneously at 15 minutes and 6 hours after ischemia respectively. For permanent MCAO model group, the occlusion thread is not drawn out and ischemia state last for 24 hours, wherein physiological saline, paeoniflorin and DPCPX+paeoniflorin are injected subcutaneously 15 minutes and 6 hours after ischemia respectively, the DPCPX is administered 15 minutes before ischemia.

Comprehensive scoring is performed according to the Syderff neurological defect physical sign evaluation method ( Sydserff SG, Borelli AR, Green AR, Cross AJ., Effect of NXY-059 on infarct volume after transient or permanent middle cerebral artery occlusion in the rat; studies on dose, plasma concentration and therapeutic time window. Br J Pharmacol. 2002; 135(1): 103-12.). Based on the degree of forelimb arcuation, spontaneous revolution and antarafacial scratch stimulus response defect, the scores of 0-2 (0=normal, 2=severe) are marked. Moreover, the torque of limbs antarafacial twist is also used as a single index with scores of 0-2 (0 = large torque, 2=small torque). Thus the injury score is between 0-8.

The experimental results indicate that the paeoniflorin of medium concentration (5mg.kg⁻¹s.c.) improves the neurological symptom of transient middle arterial occlusion rat, and significantly decreases the injury of cerebral subcortical nucleus and cerebral cortex caused by ischemia. Paeoniflorin decreases the injury of cerebral subcortical nucleus and cerebral cortex caused by ischemia dose-dependently, and this effect can be antagonized by the selective A₁R receptor antagonist DPCPX. It shows that paeoniflorin achieves the protective effects on nerves by activating the A₁R receptors, and it may be used for clinical treating and preventing of apoplexy.

### 3. The application of paeoinflorin in preventing and treating Parkinson's disease (PD):

The mouse PD model induced by 1-methyl-4-phenyl-1,2,3,6-tetrahydro-pyridine (MPTP) is used to test the effect of paeoniflorin on the ability of climbing pole and the deletion of dopamine neuron of substantia nigra of midbrain in the PD model mouse. Male C57B1/6J mice of 20-25g are used. The climbing pole time of the mice are measured before experiment. The mice are divided into 5 groups according to the climbing pole time and there are 8 mice in each group. Low dose and middle dose paeoniflorin are administered by subcutaneous injection for 11 days continuously, and 1-methyl-4-phenyl-1,2,3,6-tetrahydro-pyridine (MPTP) is injected intraperitoneally (ip) at 2 hours intervals for 4 times on day 8 of the experiment to induce mouse PD model.

The motor activity of the mice are measured by pole test, the mice are put on to the vertical coarse pole of 55cm long and 8mm diameter with head downward, and the time that the mice go downward to all the limbs contact the ground is measured (Hamaue N, Minami M, Terado M, Hirafuji M, Endo T, Machida M, Hiroshige T, Ogata A, Tashiro K, Saito H, Parvez SH. Comparative study of the effects of isatin, an endogenous MAO-inhibitor, and selegiline on bradykinesia and dopamine levels in a rat model of Parkinson's disease induced by the Japanese encephalitis virus. Neurotoxicology. 2004 Jan;25(1-2):205-13.).

Experiment results indicate that the mice in middle dose paeoniflorin group (5mg.kg⁻¹s.c.) significantly improve the motor dysfunction caused by MPTP, and antagonize the deletion of dopaminergic neuron induced by MPTP. It shows that paeoniflorin may be used in clinical treating and preventing of Parkinson's disease.

### 1. The effect of paeoniflorin to the blood pressure of conscious rat:

The conscious rats are used as research subject, sensors of tail sleeve are used to transduce the pressure pulse to phototriodes and convert it into corresponding blood pressure and heart rate values. When the rats calm down, their blood pressure and heart rate values are recorded as the base values. Then, physiological saline, various doses of paeoniflorin and reserpine are injected through caudal vein respectively.

The changes of blood pressure and heart rate before the administration (0 min) and after the administration (5min to 2hr) are recorded.

The experiment results indicate that all doses of paeoniflorin (10, 40, 160mg kg ⁻¹, i.v.) do not affect the blood pressure and heart rate of the rats. It is obvious that it has no significant cardiovascular side effects in the therapeutic dose ranges for treating and preventing nervous system diseases, such as apoplexy and Parkinson's disease.

### Description of the Drawing

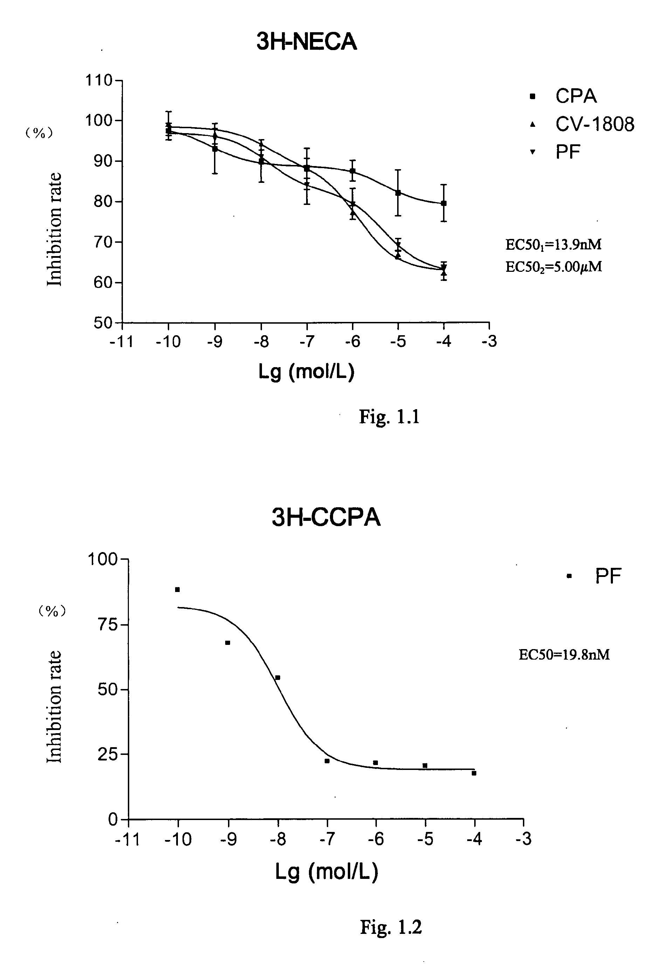

Fig. 1.1 is a graph which shows the competitive binding of various concentrations of paeoinflorin, CPA (selective A₁R receptor agonist) and CV-1808 (selective A_{2A}Receptor agonist) with [3H]-NECA (A₁R and A_{2A}R mixed agonist) to the binding sites of cerebral cortex synaptic membrane of rat. The results show that paeoniflorin dose-dependently competes the binding sites on the cerebral cortex synaptic membrane of rat with [3H]-NECA, and has the two sites competitive property (EC50₁=13.9nM, EC50₂=5.000µM).
Fig. 1.2 is a graph which shows the competitive binding of various concentration of paeoinflorin with [3H]-CCPA (selective A₁R receptor agonist) to the binding sites of cerebral cortex synaptic membrane of rat. The results show that paeoniflorin dose-dependently competes the binding sites on the cerebral cortex synaptic membrane of rat with [3H]-CCPA (EC50=19.8nM).

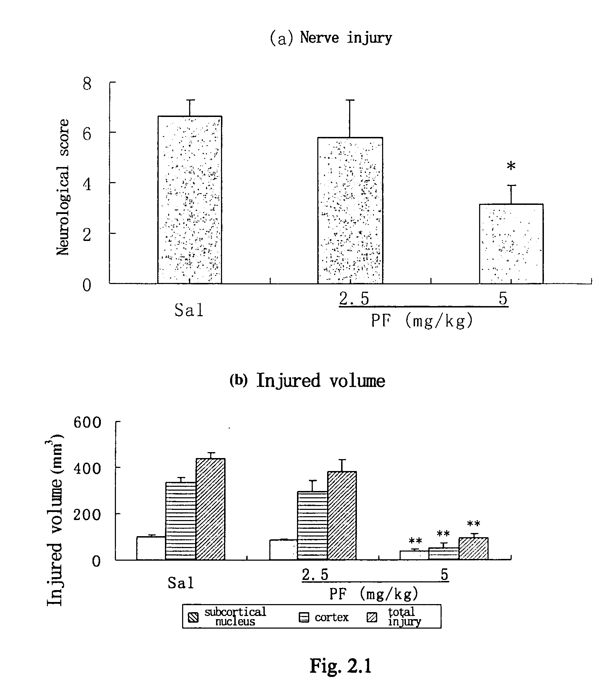

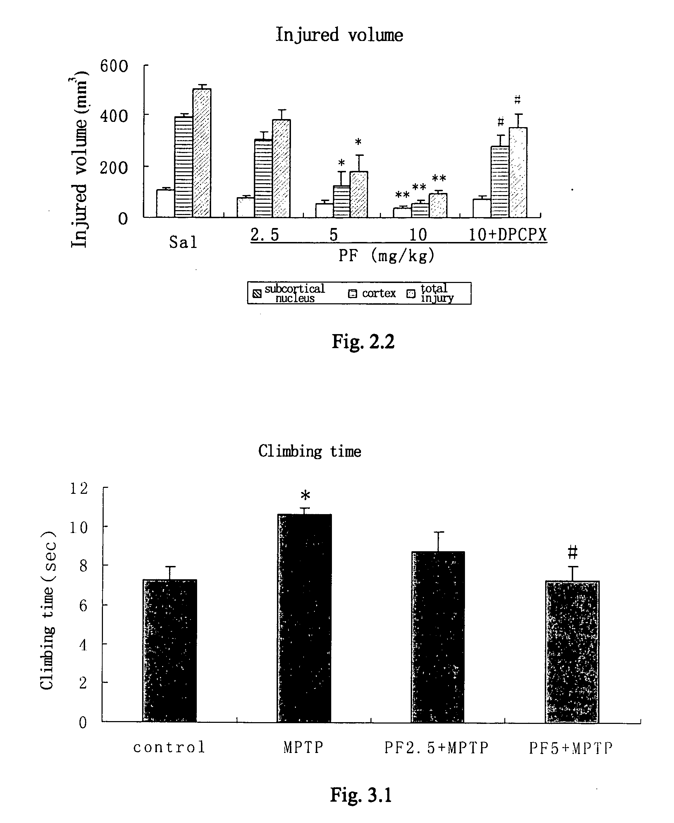

Fig. 2.1 (a) The improvement of paeoniflorin (2.5 mg·kg⁻¹, 5 mg·kg⁻¹) to transient MCACO rat neurological symptoms (n=6, *p<0.05, **p<0.01 *vs* Sal); (b) The protection of paeoniflorin (2.5 mg·kg⁻¹, 5 mg·kg⁻¹) to transient MCACO rat subcortical nucleus and cortex (n=6, *p<0.05, **p<0.01 *vs* Sal).
Fig. 2.2 The protection of paeoniflorin (2.5 mg·kg⁻¹, 5 mg·kg⁻¹, 10 mg·kg⁻¹ s.c.) on transient MCACO rat cortical nucleus and cortex, DPCPX (0.25 mg·kg⁻¹ s.c.) block the protection of paeoniflorin (n=6, * p<0.05, ** p<0.01 *vs* Sal; # p<0.05 *vs* pseoniflorin 10 mg·kg⁻¹ s.c.).

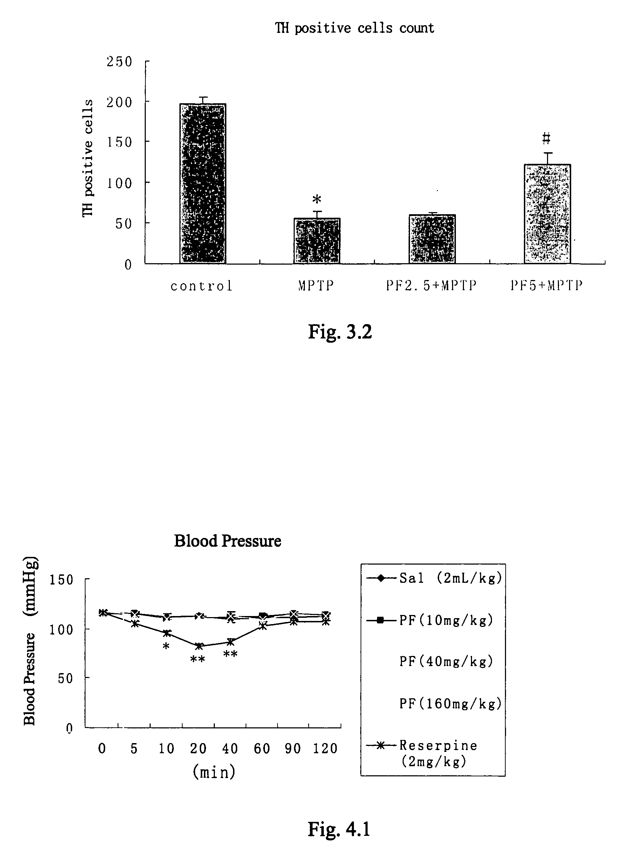

Fig. 3.1 The effect of paeoniflorin (PF) (2.5 mg·kg⁻¹, 5 mg·kg⁻¹ s.c.) on bradykinesia of the mice caused by MPTP.
Fig. 3.2 The effect of paeoniflorin (PF) (2.5 mg·kg⁻¹, 5 mg·kg⁻¹ s.c.) on the deletion of dopamineric neuron and decrease of corpus striatum dopamine neurofibra caused by MPTP.

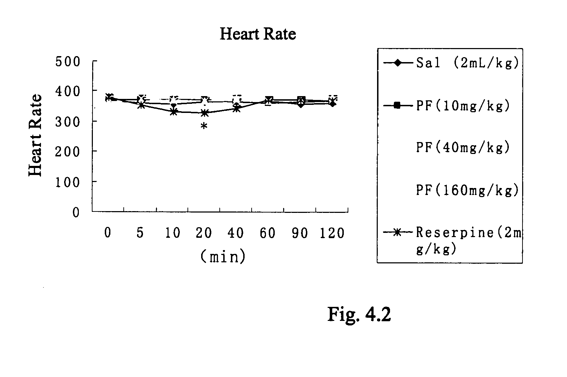

Fig. 4.1 The effect of paeoniflorin (10, 40, 160mg kg⁻¹, i.v.) does not affect the blood pressures of conscious rats (n=6; *p<0.05, **p<0.01).
Fig. 4.2 The effect of paeoniflorin (10, 40, 160mg kg⁻¹, i.v.) does not affect the heart rates of conscious rats (n=6; *p<0.05, **p<0.01).

It can be seen from the above experiment that the compound paeoniflorin provided by the invention competively binds A₁R receptor, and has high selectivity. In the case of its chemical structure, it is different from the traditional A₁R receptor agonists, and it is a new non-purine A₁R receptor agonist. By relative animal experiment identification, the paeoniflorin has the role of treating and preventing nervous system diseases such as apoplexy and Parkinson's disease etc. Further research proves that its cardiovascular side effects are small, and has clinical application value, and can be applied for treating and preventing nervous system diseases such as apoplexy and Parkinson's disease etc.

### Best Mode for Carrying out the Invention

### Example 1: Paeoniflorin is a selective agonist of a non-purine A₁ receptor

### 1.1. Preparation of synaptic membranes of rat cerebral cortex

After decapitation of the rat, cerebral cortex was separated immediately, and put it into ice-cold physiological saline. Then it was homogenized sufficiently in 1: 15 (W/V) sucrose solution, after centrifuged (1,000×g, 10min), the supernatant was taken and centrifuged (30,000×g, 30min) again. After discarding the supernatant, the sediment was resuspended in 1: 30 (W/V) Tris-HCl buffer (50 mM, pH 7.4), homogenized, washed and centrifuged (48,000xg, 10min) again, after repeating the wash process 3 times, the sediment was resuspended in 1: 5 (W/V) Tris-HCl buffer (50 mM, pH 7.4) again, and subpakaged and stored at -80 °C . The protein concentration was measured by Bradford method. All the above procedures were performed at 4°C .

### 1.2. Competitive binding of ligand-receptor

A₁R and A₂ₐR receptor mixed agonist [3H]-NECA (NEN; 15.3 Ci/mmol=0.57 TBq/mmol) and A₁R receptor selective agonist [3H]-CCPA (NEN; 30 Ci/mmol=2.9 PBq/ mol) were selected to evaluate the binding of paeoniflorin and A₁R and A₂ₐR receptor. The reaction system was 200µL, in which the drug to be tested was 5µL, which was paeoniflorin, A₁R selective agonist (CPA) and A₂ₐR receptor selective agonist (CV-1808) respectively, all final concentrations were from 0, 10⁻¹¹ to 10⁻⁴ mol /L; The cerebral cortex synaptic membrane suspension was 20µL; 25nmol/L [3H]-NECA or 0.2nmol/L [3H]-CCPA was 20µL; The analysis buffer (50 mmol/L Tris-HCl, 1 mmol EDTA, 0.5% BSA, pH 7.4) was 155µL. After the samples were incubated at 25 °C for 3 hours, the cell harvestor was used to aspirate the reaction solution, made it permeate the GF/C filter membrane, to stop the reaction, the filtration buffer (50 mmol/ L Tris-HCl, 0.1% BSA, pH 7.4) was used to wash the filter membrane for 3 times, and dried for 1 hour at 40 °C. The filter membrane was packaged into a sealed bag, and 5mL MicroScint 20 was added, pressed even and a sealing tape was added, radiation activity count (Micro β, PerkinElmer) was performed. The statistical results were fitted according to two site competitive model.

### 1.3. Experimental results

Paeoniflorin could establish a competitive binding with [3H]-CCPA, and its EC50=19.8nM; and its competitive binding with the [3H]-NECA showed a typical two-site competitive property (EC50₁=13.9nM, EC50₂=5.00µM). Result indicated that the paeoniflorin bound A₁R and A_{2A}R receptors simultaneously with a higher selectivity (EC50₂/ EC50₁=360 times), that is, it selectively bound to A₁R at low concentration and bound to A_{2A}R at high concentration.

### Example 2: Application of paeoniflorin in preventing and treating of apoplexy

### 2.1. Animal model and administration method

The transient and permanent focal cerebral ischemia model was obtained respectively by the method of reversible middle artery occlusion in rat. The rat was anesthetized by intraperitoneal injection of 350mg · kg⁻¹ chloralosane, the blood pressure was monitored by a right femoral arterial cannula, and the rectal temperature remained 36.5-37.5°C. The cervical part was cut to dissociate the external carotid artery, branch arteries were severed by electric coagulation, and the distal end is ligated, a monofilament nylon thread of No. 4-0 with the drumstick-like head was inserted from external carotid artery into the anterior cerebral artery via internal carotid artery to block the initial section of the middle cerebral artery and also block the anterior cerebral artery, the posterior cerebral artery and the branch arteries of the internal carotid artery. For the transient MCAO model group, occlusion thread was drawn out after 1.5 hours, and perfusion is performed for 22.5 hours, wherein physiological saline (2 mL·kg⁻¹) and paeoniflorin (2.5 mg·kg⁻¹, 5 mg·kg⁻¹) were injected subcutaneously at 15 minutes and 6 hours after ischemia respectively; For permanent MCAO model group, the occlusion thread was not drawn out and ischemia state lasted for 24 hours, wherein physiological saline (2 mL. kg⁻¹), paeoniflorin (2.5 mg·kg⁻¹, 5 mg·kg⁻¹, 10 mg·kg⁻¹) and DPCPX (2.5 mg·kg⁻¹)+paeoniflorin (10 mg·kg⁻¹) were injected subcutaneously 15 minutes and 6 hours after ischemia respectively, the DPCPX was administered 15 minutes before ischemia.

### 2.2. Measurement of the index

### 2.2.1. Evaluation standard of neurological defect

Comprehensive scoring was performed according to Syderff neurological defect physical sign evaluation method. Based on the degree of forelimb arcuation, spontaneous revolution and antarafacial scratch stimulus response defect, the scores of 0-2 (0=normal, 2=severe) were marked. Moreover, the torque of limbs antarafacial twist was also used as a single index with scores of 0-2 (0= large torque, 2 = small torque). Thus the defect score was between 0-8.

### 2.2.2. Functional enzymohistochemistry staining of cerebral ischemia-TTC staining

After decapitation of the rat, cerebrum was separated immediately, and stored at -20 °C for 15min, coronal section was performed continuously (section thick 2 mm). The brain sections were stained in 1%TTC (pH7.2), and incubated at 37 °C for 10min, the staining solution was discarded and the sections were fixed in 4 % formaldehyde solution. Photographs were taken by digital camera, pinel point scan (Adobe ImageReady 7.0) was carried out for the cortex and subcortex injured region of the brain sections, the area percentage of cortex injured region, subcortex injured region and total injured region was calculated, and further referring to the whole brain volume, the volume of cortex injured region, subcortex injured region and total injured region was calculated.

### 2.3. Experimental results

Experimental results indicated that the paeoniflorin (5 mg·kg⁻¹s.c.) obviously improved the neurological symptom of transient MCAO rats and significantly decreased the defect of cerebral subcotical nucleus and cerebral cortex caused by ischemia; Paeoniflorin (2.5, 5, 10 mg·kg⁻¹s.c.) dose-dependently decreased the defect of cerebral subcortical nucleus and cerebral cortex caused by ischemia and this effect could be inhibited by selective A₁R receptor antagonist DPCPX. It was suggested that paeoniflorin could has protective effects on nerves through activating A₁R receptor, and be used for clinical treating and preventing apoplexy.

### Example 3: The application of paeoinflorin for preventing and treating Parkinson's disease

### 3.1. Animal model and administration method

Male C57B1/6J mice of 20-25g were used. The climbing pole time of the mice was measured before experiment. The mice were divided into 5 groups according to the climbing pole time and there were 8 mice in each group. Paeoniflorin (2.5mg kg⁻¹, 5mg kg⁻¹) were administered by subcutaneous injection for 11 days continuously, and 1-methyl-4-phenyl-1,2,3,6-tetrahydro-pyridine (MPTP) was injected intraperitoneally (ip) at 2 hours intervals for 4 times on day 8 of the experiment to induce mouse PD model. The administration schedule was as follows:

| group | administration | dose | ip MPTP (on day 8) |
|---|---|---|---|
| Control group | Saline | 0.1 ml/10g | |
| Model group | Saline | 0.1ml/10g | 4 x 20mg/kg·d |
| Low dose group | Paeoniflorin | 2.5mg/kg | 4 x 20mg/kg•d |
| Middle dose group | Paeoniflorin | 5mg/kg | 4 x 20mg/kg•d |

### 3.2. Measurement of the index

### 3.2.1 Pole test to measure motor activity of the mice

The mice were put on to the vertical coarse pole of 55cm long and 8mm diameter with head downward, and the time that the mice went downward to all the limbs contacted the ground was measured.

### 3.2.2 Immunoflurescence staining of dopamine neuron of substantia nigra of midbrain- Tyrosine hydroxylase (TH) staining

The heart was perfused with 4% paraformaldehyde to be pro-fixed, and then the cerebrum was taken out, and post-fixed with 4% formaldehyde solution for 1 week. Coronal sections were performed continuously (section thick 30 mm). Tyrosine hydroxylase (TH) was used as the specific marker of dopamine neuron. The primary antibody was monoclonal mouse TH antibody (1:1000, Sigma), the second antibody was Alexa fluor 488 fluorescence labeled goat anti-mouse (1:1000, Molecular Probes). Photographs were taken under fluorescence microscope (Olympus DP70), and TH positive cells of substantia nigra were counted.

### 3.3.3 Experiment results

Paeoniflorin (5mg.kg⁻¹s.c.) significantly antagonized the deletion of dopaminergic neuron and motor dysfunction induced by MPTP, which suggested that paeoniflorin could be used for clinical treating and preventing Parkinson's disease.

### Example 4: Paeoniflorin did not affect the blood pressure of conscious rat:

### 4.1. Animal model and administration method

The conscious rats were used as research subject, sensor of tail sleeve were used to transduce the pressure pulse to phototriodes and converted it into corresponding blood pressure value and heart rate values. When the rats calmed down, their blood pressure and heart rate value were recorded as the base values. Then, physiological saline, paeoniflorin (10, 40, 160mg kg⁻¹) and reserpine were injected through caudal vein respectively.

### 4.2. Measurement of the index

The changes of blood pressure and heart rate before the administration (0 min) and after the administration (5min to 2hr) were recorded.

### 4.3. Experimental results

Paeoniflorin (10, 40, 160mg kg⁻¹, i.v.) did not affect the blood pressure and heart rate of the rats, it was obvious that it had no significant cardiovascular side effects in the therapeutic dose ranges for treating and preventing nervous system diseases such as apoplexy and Parkinson's disease.

## Claims

1. A medical use of paeoniflorin extracted and isolated from the roots of Panunculacease plants white peony, red peony and peony and having the following structure 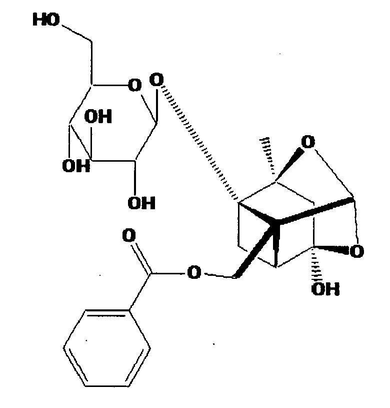 as a selective agonist of non-purine A₁ receptor.

2. The medical use of paeoniflorin according to claim 1, wherein the said use is for preparing the drugs of treating and preventing nervous system diseases.

3. The medical use of paeoniflorin according to claim 2, wherein the said use is for preparing the drugs of treating and preventing apoplexy and apoplexy sequelae.

4. The medical use of paeoniflorin according to claim 2, wherein the said use is for preparing the drugs of treating and preventing Parkinson's disease and other nervous degenerative diseases.
